# EUROPÄISCHE PATENTSCHRIFT

(11) **EP 2 194 868 B1**
(45) Veröffentlichungstag und Bekanntmachung des Hinweises auf die Patenterteilung: **20.06.2012**
(21) Anmeldenummer: 08775343.0
(22) Anmeldetag: 24.07.2008
(51) Int. Cl.: A61B 5/083, A61B 5/22

(54) **VORRICHTUNG ZUR STEUERUNG UND/ODER REGELUNG EINER TRAININGS- UND/ODER REHABILITATIONSEINHEIT**
DEVICE FOR CONTROLLING AND/OR REGULATING A TRAINING AND/OR REHABILITATION UNIT
DISPOSITIF POUR COMMANDER ET/OU RÉGULER UNE UNITÉ D'ENTRAÎNEMENT PHYSIQUE ET/OU DE RÉÉDUCATION

(30) Priorität: 18.08.2007 DE 102007039124
(43) Veröffentlichungstag der Anmeldung: 16.06.2010
(73) Patentinhaber: Jerichow, Ulrich, 63571 Gelnhausen (DE)
(72) Erfinder: Jerichow, Ulrich, 63571 Gelnhausen (DE)
(74) Vertreter: Metten, Karl-Heinz
(86) Internationale Anmeldenummer: PCT/EP2008/059737
(87) Internationale Veröffentlichungsnummer: WO 2009/024427

(56) Entgegenhaltungen:
- EP-A- 0 176 277
- EP-A- 1 825 888
- DE-U1-202006 011 058
- US-A1- 2003 013 072
- S. FASOULAS: "Solid State Electrolyte Sensors for the Determination of Oxygen, Carbon Dioxide, and Total Flow Rates Associated to Respiration in Human Subjects" INTERNET ARTICLE, [Online] 13. Oktober 2006 (2006-10-13), XP002500809 Gefunden im Internet: URL:http://www.ibtk.de/project/rss/PRO3-FR -Exec-Sum_17102006.pdf> [gefunden am 2008-10-22] in der Anmeldung erwähnt
- 'TU Dresden Portrait Dr.-Ing. Tino Schmiel', [Online] Gefunden im Internet: <URL:http://tu-dresden.de/die_tu_dresden/fa kultaeten/fakultaet_maschinenwesen/ilr/rsn/ staff/document.2010-09-08.3368629051> [gefunden am 2010-11-24]
- S. FASOULAS ET AL.: "A Miniaturised Respiratory Sensor System", MICROGRAVITY APPLICATIONS PROGRAMME: SUCCESSFUL TEAMING OF SCIENCE AND INDUSTRY, October 2005 (2005-10), pages 380-387, XP009141644, ISBN: 92-9092-971-5
- "Publications", ESA BULLETIN, vol. 124, November 2005 (2005-11), pages 96-100,

## Beschreibung

Die vorliegende Erfindung betrifft eine Vorrichtung zur Steuerung und/oder Regelung einer Trainings- und/oder Rehabilitationseinheit.

Es ist bekannt, ein Höhentraining zur Steigerung der konditionellen Leistungsfähigkeit, beispielsweise eines Sportlers, durchzuführen. Dabei wird ein entsprechender Trainingseffekt durch längere Trainingsaufenthalte in Hochgebirgsregionen erreicht. Eine exakt steuerbare und kontrollierte Leistungssteigerung ist bei einem solchen Höhentraining allerdings nicht möglich.

Für eine genaue Untersuchung der Lungenfunktion kann das Verfahren der Spirometrie angewendet werden. So wird beispielsweise in der DE 6 912 241 U eine Spirometrie-Atemmaske zur Untersuchung der Lungenfunktion, insbesondere bei körperlicher Belastung, beschrieben. Weiterhin kann gemäss der US 2006201507 eine Spirometrie-Vorrichtung für die Messung der Sauerstoffaufnahme genutzt werden. Mit solchen Systemen ist es allerdings nicht möglich, gleichzeitig die Sauerstoff- und Kohlendioxidzusammensetzung der Inspirationsluft sowie der Expirationsluft zu überwachen.

Aus Publications, ESE BULLETIN, Band 124, November 2005, Seiten 96-100 ist eine beheizte Sensoreinheit bekannt.

Aus US 2003/013072 A1 ist eine Trainingsvorrichtung bekannt, mit der oder Sauerstoffverbrauch und die Kohlendioxidproduktion erfassbar ist.

Um das Ausdauertraining bei gleichzeitiger Überwachung der Leistungsdaten sowie der Körperfunktionen für einen Sportler angenehmer zu gestalten, ist eine Integration der Trainingseinrichtung in ein System zur Bereitstellung einer virtuellen Realität möglich. Gemäß der DE 20 2004 007 273 U1 ist bereits eine Erholungs- und/oder Erlebniseinrichtung, insbesondere Wellness-Anlage, mit Filmpräsentation mittels einer Filmvorfuhreinrichtung und einem Filmabbildungsbereicht innerhalb geschlossener Räume beschrieben, wobei diese über eine Steuereinrichtung miteinander verbundene Bewegungs-, Ton-, Wind-, Geruchs-, Lichtund/oder sonstige Strahlungs-Erzeugungseinrichtungen und/oder Sensoreinrichtungen enthält, deren Aktivitäten und Funktionsweisen auf Ereignisse der Filmpräsentation zeitlich, örtlich und/oder in ihren Intensitäten abgestimmt sind. Dabei erfolgt bei solchen und ähnlichen Systemen aber keine Aufnahme und Analyse der Körperfunktionen, wie der Lungenfunktion, so dass auch keine Kontrolle über die Wirkung oder den Erfolg der Anwendung möglich ist. Weiterhin können mit solchen Systemen keine speziellen Fitness- bzw. Trainingsprogramme und auch keine medizinischen Anwendungen durchgeführt werden.

Die Aufgabe der vorliegenden Erfindung besteht darin, ein verbessertes System zur Verfügung zu stellen, mit dem eine Person beispielsweise spezielle Fitness- oder Rehabilitationsprogramme absolvieren kann, wobei die dazu verwendete Training-und/oder Rehabilitationseinheit in Abhängigkeit der Atemgaszusammensetzung des Benutzers gesteuert und/oder geregelt werden kann.

Die Aufgabe wurde durch eine Vorrichtung gemäß den Merkmalen des Patentanspruchs 1 gelöst.

Ein entscheidender Vorteil einer solchen Vorrichtung ergibt sich durch die Möglichkeit, die Trainings- und/oder Rehabilitationseinheit mit Hilfe einer steuer- und/oder regelbaren Widerstands- und/oder Bremsanordnung in Abhängigkeit der von der Sensor-Einheit jeweils getrennt bzw. zuordenbar für die Inspirations- und auch die Expirationsluft ermittelten Sauerstoff- und/oder Kohlendioxidkonzentration und des Atemflussvolumens gesteuert und/oder geregelt werden kann. Das heißt, es kann ein angestrebter Parameter der Atemgaszusammensetzung des Benutzers dadurch erreicht werden, dass mit Hilfe der steuer- und/oder regelbaren Widerstands- und/oder Bremsanordnung die Trainingsbelastung des Benutzers entsprechend verändert wird. Ein weiterer Vorteil ergibt sich dadurch, dass bei einem Trainingsvorgang die Lungenfunktion einer die Vorrichtung nutzenden Person exakt überwacht werden kann. Weiterhin kann beispielsweise eine Unterversorgung mit Sauerstoff sofort erkannt werden und das Training entsprechend modifiziert werden. Außerdem ist ein aufwendiges Kalibrieren, wie es bei Spirometrie-Vorrichtungen erforderlich ist, nicht mehr notwendig.

Bei einer vorteilhaften Ausführungsvariante ist die Sensoreinheit unmittelbar in einem von der Inspirations- und Expirationsluft der Person durchströmten Bauteil angeordnet. So kann die Sensoreinheit beispielsweise in einer Atemmaske, welche von einer Person getragen wird, eingebaut sein. Diese Anordnung hat den besonderen Vorteil, dass ein extrem geringes Totvolumen vorhanden ist.

Erfindungsgemäß werden ein Sauerstoffsensor verwendet, der zur selektiven Leitung von Sauerstoff-Ionen Yttrium-dotiertes Zirkonoxid als Elektrolyt zwischen zwei Elektroden sowie ein Trägerelement und ein Heizelement enthält sowie als Kohlendioxidsensor, der einen Elektrolyt aus einem superschnellen Natrium-Ionenleiter, zwei Elektroden, ein Trägerelement und ein Heizelement enthält (1). Der genannte superschnelle Natrium-Ionenleiter, auch NASICON genannt, kann durch die Formel Na₃-ₓZr₂(PO₄)₁₊ₓ(SiO₄)₂₋ₓ) beschrieben werden (2). Sensoren dieser Art haben den Vorteil, dass sie besonders klein und leicht sowie kostengünstig hergestellt werden können. So können für derartige Sensoren beispielsweise Abmaße von 20 x 3,5 x 0,5 mm erreicht werden (1). Solche miniaturisierten Sensoren sind damit für einen Einbau in eine Atemmaske besonders geeignet.

Für eine Messung der Sauerstoff-Sättigung des Blutes ist es von Vorteil, wenn ein Ohr-Clip in die Vorrichtung integriert ist. Außerdem kann die Vorrichtung ein Ohr-Clip für eine Messung des Pulses des Benutzers enthalten. Mit der Vorrichtung können somit umfangreiche Leistungsdaten erfasst sowie weitere medizinische Kenngrößen des Nutzers, wie beispielsweise der Herzfrequenz, aufgezeichnet werden. Die erhaltenen Messdaten können in vorteilhafter Weise mit Hilfe eines angeschlossenen Personal Digital Assistant (PDA) aufgezeichnet werden.

Die Trainings- und/oder Rehabilitationseinheit kann beispielsweise ein Ergometer, Fitnessgerät, Crosstrainer, Ruderergometer, Rudergerät, Laufband, Walker-Gerät, Spin-Bike oder ein Fahrrad sein. Die Widerstands- und/oder Bremsanordnung der Trainings- und/oder Rehabilitationseinheit kann beispielsweise eine pneumatische, hydraulische, mechanische, elektromagnetische Bremse, eine Wirbelstrom- oder Bandbremse beinhalten. Eine Trainings- und/oder Rehabilitationseinheit kann somit beispielsweise aus einem Rahmen, einem Mittel zur Kraftaufnahme, wie Pedale, einem Antriebsübertragungssystem, einem Rotationselement und einer Widerstands-und/oder Bremsanordnung umfassen. Dabei haben insbesondere magnetische bzw. elektrische Wirbelstrombremsen den Vorteil, dass sie einfach angesteuert werden können und wenig verschleißanfällig sind.

Weiterhin kann die Vorrichtung bei vorteilhafter Ausgestaltung Mittel zur zwei- und dreidimensionalen visuellen Darstellung, mindestens ein akustisches Ausgabe-und/oder Aufnahmemittel und Mittel zur Erzeugung von Wind- Temperatur und/oder Geruch umfassen. Weiterhin kann die Vorrichtung ein Mittel für die Stimulation des Tastsinns enthalten. Ferner ist es von Vorteil, wenn die Komponenten der Trainings-und/oder Rehabilitationseinheit, einschließlich der steuer- und/oder regelbaren Widerstands- und/oder Bremsanordnung, der Sensor-Einheit und der Steuereinheit für die Sensoren über ein Computersystem miteinander verbunden sind sowie über ein solches Computersystem gesteuert und/oder ausgelesen werden. Dabei kann das Computersystem mindestens aus einem Steuercomputer mit einer Benutzeroberfläche bestehen.

Bei einer vorteilhaften Ausführungsvariante der Vorrichtung ist an den Steuercomputer ein Netzwerkrechner zur Bildberechnung für das rechte und linke Auge angeschlossen. Die dabei generierten Signale können an einen auf dem Kopf des Nutzers getragenen Helm mit LCDs zur Erzeugung einer virtuellen Umgebung (Head Mounted Display HMD) weitergeleitet werden. Alternativ können die generierten Signale auch für eine Stereoproduktion zur Erzeugung einer dreidimensionalen Darstellung auf einer Leinwand genutzt werden. Vorteilhaft ist es weiterhin, wenn der Steuercomputer mit einem oder mehreren Eingabegeräten mit mindestens sechs Freiheitsgraden zur Bestimmung der Position und Orientierung verbunden ist und die Eingabegeräte wahlweise mit einer oder mehreren Tasten ausgestattet sind. Vorteilhaft ist es ferner, dass beispielsweise isometrische, isotone und/oder elastische Eingabegeräte an den Steuercomputer angeschlossen sind, wobei mit diesen Eingabegeräten beispielsweise eine Blickbewegungserfassung, Körperbewegungserfassung, Kopfbewegungserfassung und/oder Positionsbestimmung erfolgen kann. Bei einer weiteren vorteilhaften Ausgestaltung kann mit den Eingabegeräten Gestik, Mimik und/oder Sprache erfasst werden. Somit wird eine Kombination aus physischen und psychischen Reizen ermöglicht und eine Aromaanwendung oder ein Höhentraining in einem virtuellen dreidimensionalen Umfeld durchführbar.

In einer besonders vorteilhaften Ausführungsvariante wird als Eingabegerät beispielsweise ein Head Traker verwendet, der auch an dem auf dem Kopf des Nutzers getragenen Helm mit LCDs zur Erzeugung der virtuellen Umgebung (Head Mounted Display HMD) befestigt sein kann. Vorteilhaft ist es ferner, dass die visuelle Darstellungseinheit ein nicht bewegtes Bild, einen bewegten oder nicht bewegten Gegenstand, eine Computergrafik und/oder zwei- und/oder dreidimensional bewegte Bilder oder Filme wiedergibt. Es können dazu auch konventionelle Monitore für die zweidimensionale Darstellung verwendet werden.

Bei einer vorteilhaften Ausgestaltung kann die visuelle Darstellungseinheit ein Bild mit einem Sichtwinkel von 0 bis 179° wiedergeben oder für die Verwendung des Systems in den Bereichen Fitness, Wellness oder Medizin auch ein Bild mit einem Sichtwinkel von 180° oder mehr als 180° wiedergeben, wobei auch vom Benutzer vorher aufgenommene bewegte und/oder unbewegte reale Bilder dargestellt werden können.

Die akustische Ausgabeeinheit kann beispielsweise Musikinstrumente, menschliche Stimmen, Umgebungsgeräusche, wie Tierlaute, Wind, Regen, Wasserfälle, Donner und/oder Geräusche von Fahrzeugmotoren, Schüssen, Pumpen, Explosionen und/oder Erdarbeiten wiedergeben. Besonders vorteilhaft ist es, wenn Wind, Temperatur, Geruch und/oder Luftfeuchtigkeit an die dargestellte Situation in der virtuellen Realität angepasst werden können.

Weiterhin ist es von Vorteil, wenn über eine Kommunikationseinheit Anweisungen und/oder Hinweise an den Benutzer der Vorrichtung gegeben werden können und der Benutzer über eine Kommunikationseinheit mit einer die Vorrichtung beginnenden Person in Kontakt treten kann. Bei einer vorteilhaften Weiterentwicklung des Systems können durch die Entnahme von Blut auch genauere Blutbildanalysen vor, während und/oder nach der Benutzung durchgeführt werden. Beispielsweise kann mit Hilfe eines mit dem Computersystem verbundenen Zellanalysegeräts, vorzugsweise eines Geräts für die Durchflusszytometrie, die Zusammensetzung der Blutzellen exakt bestimmt werden. Auch ist unter Verwendung, vorzugsweise mit einem Fluoreszenzfarbstoff gekoppelten, spezifischen Antikörpers eine Analyse von Oberflächenmarkern auf Zellen möglich.

Ein Verfahren unter Verwendung der erfindungsgemäßen Vorrichtung zur Steuerung und/oder Regelung einer Trainingseinheit und/oder einer Rehabilitationseinheit sieht vor, dass
- eine Person eine Trainingseinheit und/oder eine Rehabilitationseinheit benutzt,
- in einer Sensor-Einheit eine Sauerstoffkonzentrationsbestimmung mit Hilfe eines beheizbaren elektrochemischen Festelektrolyt-Sensors und eine Kohlendioxid-Konzentrationsbestimmung mit Hilfe eines weiteren beheizbaren elektrochemischen Festelektrolyt-Sensors erfolgt,
- eine vom Atemflussvolumen der Person abhängige Steuerung der Heizkraft von Heizelementen der Sensoren zur Aufrechterhaltung konstanter Sensor-Temperaturen mit Hilfe eines Micro-Controllers in einer Sensor-Steuereinheit erfolgt sowie
- in Abhängigkeit der von der Sensor-Einheit ermittelten Atemgaszusammensetzung und des ermittelten Atemflussvolumens der Person eine Widerstands- oder Bremsanordnung der Trainings und/oder Rehabilitationseinheit gesteuert und/oder geregelt wird.

Dieses Verfahren kann unter Verwendung der oben beschriebenen Vorrichtung in einer oder mehreren der genannten Ausführungsformen durchgeführt werden. Besonders vorteilhaft erfolgt die Bestimmung der Sauerstoffkonzentration der Atemluft durch Messung des bei konstanter Spannung durch den Elektrolyt des Sauerstoffsensors von der Kathode zur Anode fließenden Stroms, wobei ein linearer Zusammenhang zwischen dem resultierenden elektrischen Strom und der Sauerstoffkonzentration besteht. Ferner ist es von Vorteil, wenn die Kohlendioxidkonzentration über einen logarithmischen Zusammenhang zwischen der Spannung zwischen den Elektroden des Kohlendioxidsensors und der Kohlendioxidkonzentration bestimmt wird. Ferner ist es vorteilhaft, dass das Atemflussvolumen aus der durch den Mikro-Controller gesteuerten - zur Aufrechterhaltung einer konstanten Sensortemperatur notwendigen - Heizkraft der Heizelemente der Sensoren bestimmt wird.

Die Bestimmung der Gesamtflussrate der Atemluft kann mit dem Sensorelement unter Ausnutzung der Dünnschicht Anemometrie erfolgen. Weiterhin kann die Flussrichtung des Atemgases entweder durch Verwendung der gemessenen Sauerstoff-und/oder Kohlendioxidkonzentrationsgradienten oder des Temperaturprofils auf dem Sensor ermittelt werden. Das Verfahren hat den Vorteil, dass gleichzeitig der Volumenstrom, die Strömungsrichtung und somit die Sauerstoff- und Kohlendioxidzusammensetzung der Inspirationsluft sowie der Expirationsluft mit einer Atemzug-für-Atemzug Auflösung überwacht werden können. Die Sauerstoff- und Kohlendioxid-Konzentrationen können also der Inspirationsluft und der Expirationsluft eindeutig zugeordnet werden. Besonders vorteilhaft ist es dabei, wenn in Abhängigkeit der ermittelten Atemgaszusammensetzung einer die Trainings und/oder Rehabilitationseinheit benutzenden Person mit Hilfe der steuer- und/oder regelbaren Widerstands- und/oder Bremsanordnung die Trainingsbelastung der Person je nach Bedarf verändert werden kann. So kann in Abhängigkeit des ermittelten Sauerstoff-und/oder Kohlendioxidgehalts des Atemgases mit Hilfe der steuer- und/oder regelbaren Widerstands- und/oder Bremsanordnung die Trainingsbelastung der Person je nach Bedarf angepasst werden kann. Beispielsweise kann der Sauerstoff- und/oder Kohlendioxidgehalt der Expirationsluft durch eine Erhöhung der Belastung mit Hilfe der Widerstands- und/oder Bremsanordnung vermindert werden.

Somit kann die erfindungsgemäße Vorrichtung zur Steigerung der Ausdauerleistung, vorzugsweise mittels eines simulierten Höhentrainings, verwendet werden.

So kann beispielsweise eine Senkung des Sauerstoffgehalts der Expirationsluft von 17% auf 12% durch eine entsprechende Erhöhung der Trainingsbelastung erreicht werden.

Weiterhin kann durch eine individuelle Belastungsanpassung das Verhältnis (respiratorische Quotient) von inspiratorischer zu expiratorischer Luft durch die erfindungsgemäße Vorrichtung unabhängig vom Tages- oder Trainingszustand bei jedem Training oder jeder Therapie konstant gehalten werden.

Es ist ferner von Vorteil, dass ein Computerprogramm mit Programmcode zur Durchführung aller oben genannten Verfahrensschritte genutzt wird, wenn das Programm in einem Computer ausgeführt wird. Dabei ist es von Vorteil, wenn das Computerprogramm mit Programmcode zur Durchführung aller oben genannten Verfahrensschritte auf einem maschinenlesbaren Träger gespeichert ist, wenn das Programm in einem Computer ausgeführt wird.

Unter Anwendung der erfindunsgemäßen Vorrichtung können sich beispielsweise Spitzen- und Leistungssportier optimal mit Höhentrainingseinheiten im virtuellen realitätsnahen Umfeld auf anstehende Wettkämpfe vorbereiten. Das realitätsnahe Training unter sauerstoffarmen Bedingungen zielt bei Breiten- und Freizeitsportlem eher auf die Steigerung der persönlichen Leistungsfähigkeit und des individuellen Konditionsniveaus ab. Dabei können im speziellen die kosten- und zeitintensiven Flüge und Aufenthalte in Hochgebirgsregionen eingespart werden. Weiterhin ist ein wesentlich effizienteres Training möglich, da die Anlage 24 Stunden verfügbar und logistisch leicht erreichbar ist.

Im Bereich Rehabilitation oder Wellness könnte dieses System in einem virtuellen dreidimensionalen Umfeld beispielsweise eine Aromaanwendung mit einem passiven Höhentraining und einer Sauerstofftherapie kombinieren. In einer solchen Umgebung könnte eine solche Kombination aus Entspannung und Verbesserung der persönlichen Leistungsfähigkeit sowie eine Stärkung des Immunsystems erreicht werden.

Im Bereich der Medizin kann das System für eine Aromaanwendung, ein Höhentraining und/oder eine Sauerstofftherapie in einem dreidimensionalen Umfeld genutzt werden, wobei die vier Sinne Sehen, Fühlen, Riechen und Hören stimuliert werden. Durch die dabei erreichte Mobilisierung des körpereigenen Abwehrsystems ist eine Anwendung bei Personen mit Erkrankungen wie beispielsweise Krebs, Allergien und Erkrankungen des Stoffwechsels denkbar.

Weiterhin bietet besonders die Technik der dreidimensionalen Darstellung die Möglichkeit, spezielle psychische Erkrankungsverläufe, wie Ängste bei Autoimmunsystemerkrankungen, durch die Wirkung von Bildern und Geräuschen positiv zu beeinflussen.

### Literatur:

(1) R. Baumann^{1,2}, S. Fasoulas¹, M. Gläser¹, C. Gritzner¹, F. Hammer², J. Heisig¹, R. Kahle¹, T. Kirschke¹, T. Schmiel¹, M. Völkel². Solid State Electrolyte Sensors for the Determination of Oxygen, Carbon Dioxide, and Total Flow Rates Associated to Respiration in Human Subjects. ¹Institute for Aerospace Engineering, Technische Universität Dresden, 01062 Dresden, Germany ²ESCUBE GmbH, Nobelstr. 15, 70569 Stuttgart, Germany (PRO2-FR-Exec-Sum-05-02-10 Executive Summary to the ESTEC Contract No. 15450/01/NL/JS CCN 1+2)
(2) West, A. R., Grundlagen der Festkörperchemie, Verlag Chemie, Weinheim (1992).

## Patentansprüche

1. Vorrichtung zur Steuerung und/oder Regelung einer Trainings- und/oder Rehabilitationseinheit, **dadurch gekennzeichnet, dass** die Vorrichtung mindestens
- eine Trainings- und/oder Rehabilitationseinheit,
- eine Sensor-Einheit mit einem beheizbaren elektrochemischen Festelektrolyt-Sensor, der zur Sauerstoffkonzentrationsbestimmung Yttriumdotiertes Zirkonoxid als Elektrolyt, zwei Elektroden sowie ein Trägerelement und ein Heizelement, und zur Kohlendioxidkonzentrationsbestimmung einen weiteren beheizbaren elektrochemischen Festelektrolyt-Sensor enthält, der einen superschnellen Natrium-Ionenleiter als Elektrolyt, zwei Elektroden sowie ein Trägerelement und ein Heizelement aufweist,
- eine Steuereinheit für die Sensoren,
- einen Micro-Controller in der Steuereinheit für die vom Atemflussvolumen abhängige Steuerung der Heizkraft von Heizelementen der Sensoren zur Aufrechterhaltung konstanter Sensor-Temperaturen,
- eine in Abhängigkeit der von der Sensor-Einheit jeweils getrennt bzw. zuordnenbar für die Inspirations- und auch die Expirationsluft ermittelten Sauerstoff-und/oder Kohlendioxydkonzentration und des ermittelten Atemflussvolumens steuer-und/oder regelbare Widerstands- und/oder Bremsanordnung der Trainings-und/oder Rehabilitationseinheit
umfasst.

2. Vorrichtung nach Anspruch 1, **dadurch gekennzeichnet, dass** die Sensor-Einheit unmittelbar in einem von der Inspirations- und Expirationsluft einer Person durchströmten Bauteil angeordnet ist.

3. Vorrichtung nach Anspruch 1 oder 2, **dadurch gekennzeichnet, dass** ein Ohr-Clip für eine Messung der Sauerstoff-Sättigung des Blutes integriert ist und/oder ein Ohr-Clip für eine Messung des Pulses integriert ist.

4. Vorrichtung nach einem der vorangehenden Ansprüche, **dadurch gekennzeichnet, dass** mit Hilfe eines angeschlossenen Personal Digital Assistant die Mess-Daten aufzeichenbar sind.

5. Vorrichtung nach einem der vorangehenden Ansprüche, **dadurch gekennzeichnet, dass** die Widerstands- und/oder Bremsanordnung der Trainings- und/oder Rehabilitationseinheit eine pneumatische, hydraulische, mechanische, elektromagnetische Bremse, eine Wirbelstrom- oder Bandbremse beinhaltet.

6. Vorrichtung nach einem der vorangehenden Ansprüche, **dadurch gekennzeichnet, dass** das Mittel zur zwei- und/oder dreidimensionalen visuellen Darstellung, mindestens ein akustisches Ausgabe- und/oder Aufnahmemittel und Mittel zur Erzeugung von Wind, Temperatur und/oder Geruch angeschlossen sind und/oder dass Mittel für die Stimulation des Tastsinns angeschlossen sind.

7. Vorrichtung nach einem der vorangehenden Ansprüche, **dadurch gekennzeichnet, dass** die Komponenten der Trainings- und/oder Rehabilitationseinheit, einschließlich der steuer- und/oder regelbaren Widerstands- und/oder Bremsanordnung, der Sensor-Einheit und der Steuereinheit für die Sensoren über ein Computersystem miteinander verbunden sowie steuerbar und/oder auslesbar sind.

8. Vorrichtung nach einem der vorangehenden Ansprüche, **dadurch gekennzeichnet, dass** das Computersystem mindestens einen Steuercomputer mit einer Benutzeroberfläche enthält.

9. Vorrichtung nach einem der vorangehenden Ansprüche, **dadurch gekennzeichnet, dass** die generierten Signale an einen auf dem Kopf des Nutzers getragenen Helm mit LDCs zur Erzeugung der virtuellen Umgebung (Head Mounted Display-HMD) weiterleitbar sind.

10. Vorrichtung nach einem der vorangehenden Ansprüche, **dadurch gekennzeichnet, dass** die generierten Signale für eine Stereo-Projektion zur Erzeugung einer dreidimensionalen Darstellung auf einer Leinwand nutzbar sind.

11. Vorrichtung nach Anspruch 8 oder Anspruch 9, soweit dieser auf Anspruch 8 rückbezogen ist, oder Anspruch 10, soweit dieser direkt oder indirekt auf Anspruch 8 rückbezogen ist, **dadurch gekennzeichnet, dass** der Steuercomputer mit einem oder mehreren Eingabegeräten mit mindestens sechs Freiheitsgraden zur Bestimmung der Position und Orientierung verbunden ist und die Eingabegeräte wahlweise mit einer oder mehreren Tasten ausgestattet sind.

12. Vorrichtung nach Anspruch 11, **dadurch gekennzeichnet, dass** das Eingabegerät einen Head Tracker darstellt.

13. Vorrichtung nach einem der vorangehenden Ansprüche, **dadurch gekennzeichnet, dass** mit dem Computersystem ein Zellanalysegerät, vorzugsweise ein Gerät für die Durchflusszytometrie, verbunden ist.

## Claims

1. A device for controlling and/or regulating a training and/or rehabilitation unit, **characterized in that** the device comprises at least
- a training and/or rehabillitation unit,
- a sensor unit including a heatable electrochemical solid-electrolyte sensor, for the determination of the oxygen concentration, containing yttrium-doped zirconium oxide as an electolyte, two electrodes as well as a carrier element and a healing element, and another heatable electrochemical solid-electrolyte sensor, for the determination of the carbon dioxide concentration, containing a super-fast sodium ion conductor as an electrolyte, two electrodes as well as a carrier element and a heating element,
- a control unit the sensors.
- a micro-controller in the control unit for the control of the heating power of heating elements of the sensors in accordance with the breath volume to maintain constant sensor temperatures,
- a resistance and/or brake arrangement of the training and/or rehabilitation unit that can be controlled and/or regulated in accordance with the oxygen and/or carbon dioxide concentration that has been determined by the sensor unit separately for, or in relation to, the inspiration air and the expiration air, and in accordance with the breath volume.

2. A device according to claim 1, **characterized in that** the sensor unit is arranged directly in a component through which the inspiration and expiration air of person flows.

3. A device according to claim 1 or 2, **characterized in that** an ear clip for measuring the oxygen saturation of the blood is integrated, and/or an ear clip for measuring the pulse is integrated.

4. A device according to any one of the preceding claims, **characterized in that** the measured data are recordable by means of a Personal Digital Assistant connected to said device.

5. A device according to any one of the preceding claims, **characterized in that** the resistance and/or brake arrangement of the training and/or rehabilitation unit includes a pneumatic, hydraulic, mechanical, electromagnetic brake, an eddy-current brake or a band brake.

6. A device according to any one of the preceding claims, **characterized in that** a means for two- and/or three-dimensional visual representation, at least one acoustic output and/or recording means and means for producing wind, temperature and/or odour arc connected, and/or that means for stimulating the sense of touch are connected.

7. A device according to any one of the preceding claims, **characterized in that** the components of the training and/or rehabilitation unit, including the resistance and/or brake arrangement that can be controlled and/or regulated, the sensor unit and the control unit for the sensors, are interconnected and can be controlled and/or read by means of a computer system.

8. A device according to any one of the preceding claims, **characterized in that** the computer system contains at least one control computer having a user interface.

9. A device according to any one of the preceding claims, **characterized in that** the signals that are generated can he transmitted to a helmet that is worn on the head of the user and is equipped with LCDs for producing a virtual environment (head-mounted display, HMD).

10. A device according to any one of the preceding claims, **characterized in that** the signals that arc generated are usable for a stereo projection that serves to produce a three-dimensional representation on a screen.

11. A device according to claim 8 or claim 9, insofar as the latter refers back to claim 8, or claim 10, insofar it directly or indirectly refers back to claim 8, **characterized in that** the control computer is connected to one or more input devices having at least six degrees of freedom for position determination and orientation, and the input devices are optionally equipped with one or more buttons.

12. A device according to claim 11, **characterized in that** the input device is a head tracker.

13. A device according to any one of the preceding, claims, **characterized in that** a cell analysis apparatus, preferably an apparatus for flow cytometry, is connected to the computer system.

## Revendications

1. Dispositif pour commander et/ou régler une unité d'entrainement physique et/ou de rééducation, **caractérisé en ce que** le dispositif comprend au moins
- une unité d'entraînement physique et/ou de rééducation,
- une unité de capteur avec un capteur à électrolyte solide électrochimique chauffé qui, pour la détermination de la concentration en oxygène, contient de l'oxyde de zirconium dopé à l'yttrium en tant qu'électrolyte, deux électrodes ainsi qu'un élément porteur et un élément chauffant, et, pour la détermination de la concentration en dioxyde de carbone, un autre capteur à électrolyte solide électrochimique chauffé qui présente un conducteur d'ions sodium super rapide en tant qu'électrolyte, deux électrodes ainsi qu'un élément porteur et un élément chauffant,
- une unité de commande pour les capteurs,
- un microcontrôleur dans l'unité de commande pour la commande, en fonction du volume de flux respiratoire, de la force de chauffage d'éléments chauffants des capteurs pour le maintien de températures de capteur constantes.
- un dispositif de résistance et/ou de freinage de l'unité d'entraînement physique et/ou de rééducation pouvant être commandé et/ou réglé en fonction de la concentration en oxygène et/ou en dioxyde de carbone déterminée par l'unité de capteur, respectivement de façon séparée ou pouvant être affectée, pour l'air inspiré et également pour l'air expiré, et en fonction du volume de flux respiratoire déterminé.

2. Dispositif selon la revendication 1, **caractérisé en ce que** l'unité de capteur est disposée directement dans un composant parcouru par l'air inspiré et expiré d'une personne.

3. Dispositif selon la revendication 1 ou la revendication 2, **caractérisé en ce qu'**un clip auriculaire est intégré pour une mesure de la saturation en oxygène du sang et/ou **en ce qu'**un clip auriculaire est intégré pour une mesure du pouls.

4. Dispositif selon une des revendications précédentes, **caractérisé en ce que** les données de mesure peuvent être enregistrées à l'aide d'un assistant numérique personnel connecté.

5. Dispositif selon une des revendications précédentes, **caractérisé en ce que** le dispositif de résistance et/ou de freinage de l'unité d'entraînement physique et/ou de rééducation contient un frein pneumatique, hydraulique, mécanique, électromagnétique, un frein à courants de Foucault ou à ruban.

6. Dispositif selon une des revendications précédentes, **caractérisé en ce que** le moyen de représentation visuel en deux et/ou en trois dimensions, au moins un moyen de production et/ou d'enregistrement acoustique et des moyens pour la production de vent, de température et/ou d'odeur sont connectés et/ou **en ce que** des moyens pour la stimulation du sens du toucher sont connectés.

7. Dispositif selon une des revendications précédentes, **caractérisé en ce que** les composants de l'unité d'entraînement physique et/ou de rééducation, y compris le dispositif de résistance et/ou de freinage commandé et/ou réglé, l'unité de capteur et l'unité de commande pour les capteurs sont connectés les uns aux autres par le biais d'un système informatique et peuvent également être commandés et/ou appelées.

8. Dispositif selon une des revendications précédentes, **caractérisé en ce que** le système informatique continent au moins un ordinateur de commande avec une surface opérateur.

9. Dispositif selon une des revendications précédentes, **caractérisé en ce que** les signaux générés peuvent être transmis à un casque porte sur la tête de l'utilisateur avec des LCD pour la production de l'environnement virtuel (Head Mounted Display-HMD).

10. Dispositif selon une des revendications précédentes, **caractérisé en ce que** les signaux générés peuvent être utilisés pour une projection stéréo pour la production d'une représentation en trois dimensions sur un écran de projection.

11. Dispositif selon la revendication 8 ou la revendication 9 dans la mesure où celle-ci fait référence à la revendication 8, ou selon la revendication 10 dans la mesure où celle-ci fait directement ou indirectement référence à la revendication 8, **caractérisé en ce que** le système informatique est connecté à un ou plusieurs appareils d'entrée avec au moins six degrés de liberté pour la détermination de la position et de l'orientation, et **en ce que** les appareils d'entrée sont équipés au choix d'une ou de plusieurs touches.

12. Dispositif selon la revendication 11, **caractérisé en ce que** l'appareil d'entrée représente un Head Tracker.

13. Dispositif selon une des revendications précédentes, **caractérisé en ce qu'**au système informatique est connecté un appareil d'analyse cellulaire, de préférence un appareil pour la cytométrie de flux.
